Europäisches Patentamt

European Patent Office

Office européen des brevets

(19)

(11) Numéro de publication: **0 030 210 B1**

(12) **FASCICULE DE BREVET EUROPEEN**

(45) Date de publication du fascicule du brevet: **29.08.84**

(21) Numéro de dépôt: **80810357.6**

(22) Date de dépôt: **19.11.80**

(51) Int. Cl.³: **A 61 B 3/12,** A 61 B 17/36, A 61 F 9/00, B 23 K 26/02, B 23 K 26/10

(54) **Dispositif pour l'observation en vue du traitement de l'oeil.**

(30) Priorité: **28.11.79 CH 10570/79**
**05.03.80 FR 8004994**

(43) Date de publication de la demande:
**10.06.81 Bulletin 81/23**

(45) Mention de la délivrance du brevet:
**29.08.84 Bulletin 84/35**

(84) Etats contractants désignés:
**AT CH DE FR GB IT LI NL**

(56) Documents cités:
**FR-A-2 163 302**
**FR-A-2 197 614**
**GB-A- 964 567**
**GB-A-2 020 846**
**US-A-3 769 963**
**US-A-3 850 527**
**US-A-4 135 791**

(73) Titulaire: **LASAG AG**
**Bernstrasse 11**
**CH-3600 Thun (CH)**

(72) Inventeur: **Roussel, Philippe**
**14 F, Jungfraustrasse**
**CH-3600 Thun (CH)**
Inventeur: **Fankhauser, Franz, Prof.**
**Universitäts-Augenklinik Inselspital**
**CH-3010 Bern (CH)**
Inventeur: **van der Zypen, Eugen, Prof.**
**Anatomisches Institut, Universität 26,**
**Bühlstrasse**
**CH-3000 Bern (CH)**

(74) Mandataire: **Gresset, Jean et al**
**ASUAG Département Brevets et Licences**
**Faubourg du Lac 6**
**CH-2501 Bienne (CH)**

Courier Press, Leamington Spa, England.

## Description

La présente invention a trait à un dispositif pour l'observation en vue du traitement de l'oeil. Un tel dispositif, décrit par exemple dans le brevet US—A—3710 798, comporte un microscope pour observer un point de l'oeil éclairé par un lampe, un laser au $CO_2$ pour délivrer des impulsions formant un faisceau laser de traitement focalisé dans la zone d'observation au moyen d'une lentille convergente, et un laser Helium Néon délivrant un rayonnement cohérent visible qui est divisé en deux faisceaux élémentaires dont l'intersection visualise le point de focalisation du faisceau de traitement.

Un tel dispositif permet de s'assurer que le faisceau de traitement est correctement focalisé, mais il ne révèle pas à l'opérateur si un obstacle est placé sur le trajet du faisceau de traitement entre sa sortie du dispositif et le point de focalisation, surtout lorsque cet obstacle ne se trouve pas sur le trajet des deux faisceaux élémentaires visibles. Une telle configuration est très dangereuse, car elle peut conduire soit à des dégâts au niveau de l'obstacle, par exemple à l'intérieur même de l'oeil, soit à des effets optiques incontrôlés au niveau du point de focalisation du faisceau de traitement.

Le but de l'invention est donc de proposer un moyen permettant de matérialiser, de rendre visible, le faisceau de traitement dans son chemin dans l'oeil non seulement pour déterminer le plan de focalisation mais aussi pour éviter que ce faisceau ne touche un endroit non désiré.

Le dispositif pour l'observation de l'oeil selon l'invention est défini dans les revendications jointes. Il sera bien compris à la lecture de la description suivante, faite en référence aux dessins joints, parmi lesquels:

— la figure 1 est un schéma général du dispositif selon l'invention et

— les figures 2a à 2c représentent un détail de réalisation du dispositif de la figure 1.

Le faisceau optique d'observation est signalé par la zone 1. A partir de l'oculaire 2, le faisceau est réfléchi consécutivement par les miroirs 3, 4, 5, jusqu'à la lentille convergente 6 dont le foyer est au foyer de l'oculaire 2. A partir de la lentille 6, le faisceau optique d'observation est donc un faisceau, réfléchi par les miroirs 7, 8, 9, pour être amené coaxialement sur la lentille de focalisation, ou objectif 10. Le faisceau optique est focalisé par cette dernière lentille de focalisation 10, à travers le verre de contact 11, sur la zone ou point d'observation 12 de l'oeil du patient. Dans le cas présent, le point d'observation 12 est l'angle irido-cornéen.

Le faisceau optique d'éclairage 13 est issu du bloc 14 comportant la source proprement dite (lampe à incandescence, lampe à arc, à décharge, etc.) et un condenseur avec son diaphragme. Le faisceau d'éclairage 13 est rendu parallèle par une lentille convergente 15 dont le foyer est situé dans le plan du diaphragme du bloc 14. Il est ensuite réfléchi par le miroir 16 et est partagé dans l'exemple concret illustré, en deux faisceaux 13' et 13" par le diviseur de faisceau 17, par exemple, des prismes ou un miroir semi-transparent. A la suite de plusieurs réflexions sur les éléments optiques (prismes ou miroirs) 18 et 19 d'une part et 20, 21 et 22 d'autre part, les deux faisceaux parallèles 13' et 13" sont réfléchis par le miroir 9 et arrivent de manière parallèle et excentrique au faisceau optique d'observation 1 sur la lentille de focalisation 10. Les faisceaux d'éclairage 13' et 13" sont ensuite focalisés au foyer de la lentille 10, c'est-à-dire au point d'observation 12 où l'on obtient l'image du diaphragme du bloc 14.

Le dispositif selon l'invention ne se prête pas uniquement à l'observation et à l'examen de l'oeil mais également au traitement chirurgical de l'oeil au moyen de rayons laser. Une des applications principales du dispositif réside dans la possibilité de traitement chirurgical di glaucome, après observation et diagnostic du médecin.

Le faisceau laser de traitement responsable de l'opération chirurgicale est indiqué sous 23. Le faisceau laser de traitement 23 peut être issu, par exemple, d'une source laser à corps solide fonctionnant par impulsions (c.à.d. en mode Q switch). La longueur d'onde serait typiquement voisine de un micron. Un laser Nd-YAG par exemple émet principalement à $1,06\ \mu$.

Contrairement au faisceau d'observation 1 où l'on cherche une grande profondeur de champ, le faisceau de traitement laser 23 dans l'application chirurgicale recherchée doit présenter une ouverture maximale, compte tenu des contraintes physiques, afin d'obtenir de hautes densités énergétiques parfaitement localisées dans l'espace. Ainsi le faisceau est élargi par la lentille 24, rendu parallèle par la lentille 25 et focalisé par la lentille convergente 26. Le faisceau laser de traitement est superposé au faisceau optique d'observation 1 au moyen d'une surface réfléchissante 27. Cette surface peut être constituée d'un miroir ne s'interposant dans le chemin optique, par rotation ou translation, uniquement qu'au moment du tir laser 23. Néanmoins, la surface réfléchissante 27 est de préférence une lame avec couche interférentielle, réfléchissante pour une très faible bande de longueurs d'ondes centrée autour de la radiation laser de traitement considéré: par exemple la radiation $1,06\ \mu$ du Nd-YAG.

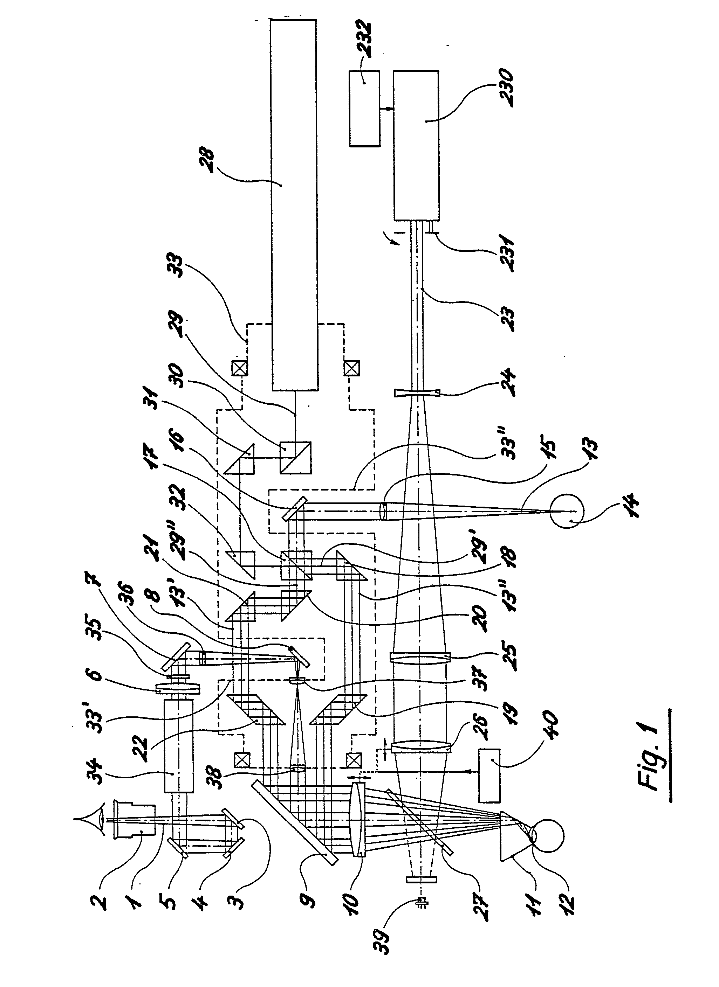

La position axiale du point de focalisation du faisceau laser de traitement doit pouvoir être définie à l'avance avec grande précision. Or, le réglage de mise au point se fait au travers de la lentille 10 comme s'il se faisait directement sur la lentille 26. Ceci tient à la particularité de l'appareil illustré en figure 1 et choisi comme exemple de réalisation, où la lentille 26 est en fait l'image de la lentille 10 donnée par le miroir 27, les deux lentilles étant accouplées par un dispositif 40, de préférence un dispositif à contrôle électronique. Tout déplacement manuel de la lentille 10 est alors transmis à la lentille 26.

Le dispositif 40 à contrôle électronique permet le déplacement des lentilles 10 et 26 selon les flèches représentées, la liaison mécanique entre les lentilles 10 et 26 et un moteur d'entraînement, non représenté afin de ne pas nuire à la clarté et à la compréhension de cette figure, étant représentée par des traits pointillés. A titre d'exemple le moteur d'entraînement peut être constitué par un moteur à courant continu, ce type de moteur permettant des commandes de déplacements de précision avec une bonne linéarité. Le système de commande de déplacement des lentilles ne sera pas décrit en détail car ces types de circuits de commande sont bien connus de l'homme de l'art.

Etant donné la grande profondeur de champ du faisceau d'observation 1, l'observation visuelle ne permet pas de situer avec précision la position du foyer de la lentille 10, et par voie de conséquence de celui de la lentille 26.

Un dispositif annexe sert à déterminer avec exactitude le plan focal de la lentille 10. Il s'agit d'une source laser continue 28 de faible puissance, émettant dans le visible, par exemple un laser He-Ne. Le faisceau laser émis 29 est dirigé au travers des surfaces réfléchissantes (prismes ou miroirs) 30, 31 et 32 sur le même diviseur de faisceau 17, d'où il se subdivise et parcourt les mêmes chemins optiques que les faisceaux d'éclairage 13' et 13". Les faisceaux lasers subdivisés 29' et 29" sont focalisés par la lentille 10 en son foyer.

Chacun des faisceaux incidents sur le tissu de l'oeil donne lieu à un spot lumineux.

Le plan focal de la lentille 10, et par conséquent le plan de travail de la lentille 26, sont parfaitement situés à la surface du tissu dans l'oeil lors de la coïncidence et de la superposition des spots lumineux observée par le médecin ophtalmologue. Ce dispositif permet, en choisissant un écart maximal entre les faisceaux 29' et 29" d'avoir une mise au point extrêmement sensible du foyer du faisceau de traitement 23 par rapport à la cible (ou point d'observation 12) et ce malgré un dispositif d'observation à grande profondeur de champ.

Cette première mise au point terminée, la position axiale du point de focalisation du faisceau laser de traitement par rapport à la surface traitée doit pouvoir être modifiable en fonction de certains paramètres (énergie notamment) soit devant, soit derrière ladite surface. Ce décalage axial entre point de focalisation du faisceau de traitement et surface traitée peut être obtenu, par exemple, par mouvement relatif des objectifs 10 et 26. Le dispositif de contrôle électronique 40 peut, dans ce cas, contrôler avec précision ledit décalage.

On remarque que l'écart donné aux faisceaux laser visibles 29', 29" à la sortie de la lentille de focalisation 10 est tel que ces faisceaux délimitent l'ouverture donnée au faisceau laser de traitement 23. En effet, il est primordial que le faisceau 23 ne puisse atteindre ni entrer en contact avec aucune autre partie de l'oeil que celle sur laquelle il est dirigé. Ceci est spécialement vrai pour l'iris qui, vu sa pigmentation serait immédiatement endommagé par des faisceaux laser à haute puissance.

Par l'aide d'un moyen essentiel de l'invention, les deux rayons 29' et 29" permettent de visualiser le point de focalisation du faisceau de traitement en étant tangents à la surface latérale externe enveloppe du faisceau laser de traitement.

A cet effet, l'ensemble des miroirs responsables de la subdivision des faisceaux 13 et 29 sont montés dans un tube 33 pouvant être tourné autour de son axe qui est en même temps l'axe optique du système. Il s'agit en l'occurence des miroirs ou prismes 17, 18, 19, 20, 21, 22, 30, 31 et 32. Le miroir 9 est à nouveau un miroir fixe. Le tube 33 comprend des ouvertures radiales 33' et 33" pour laisser passer les faisceaux d'observation 1 et d'éclairage 13 qui s'introduisent transversalement dans le dispositif optique.

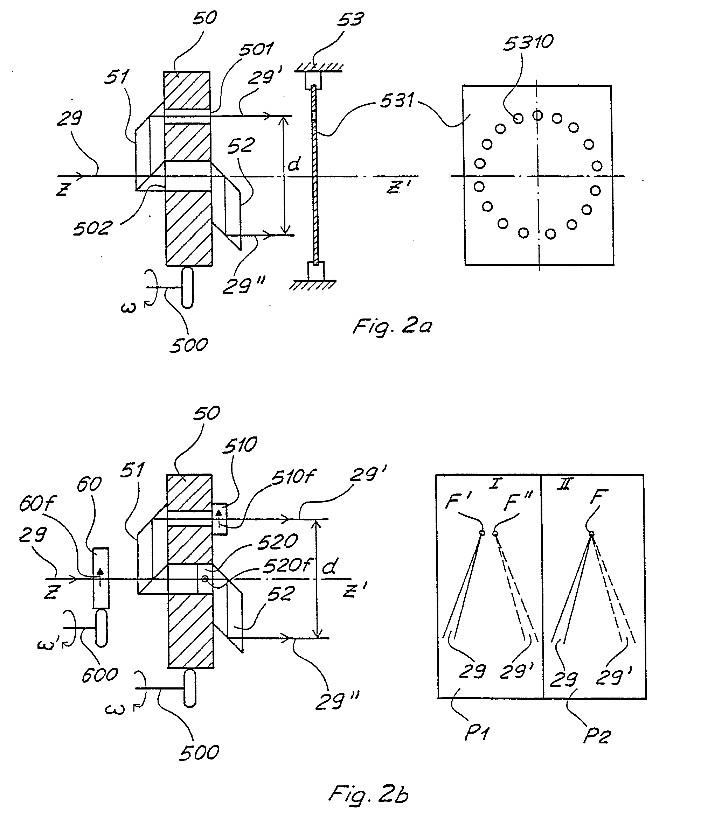

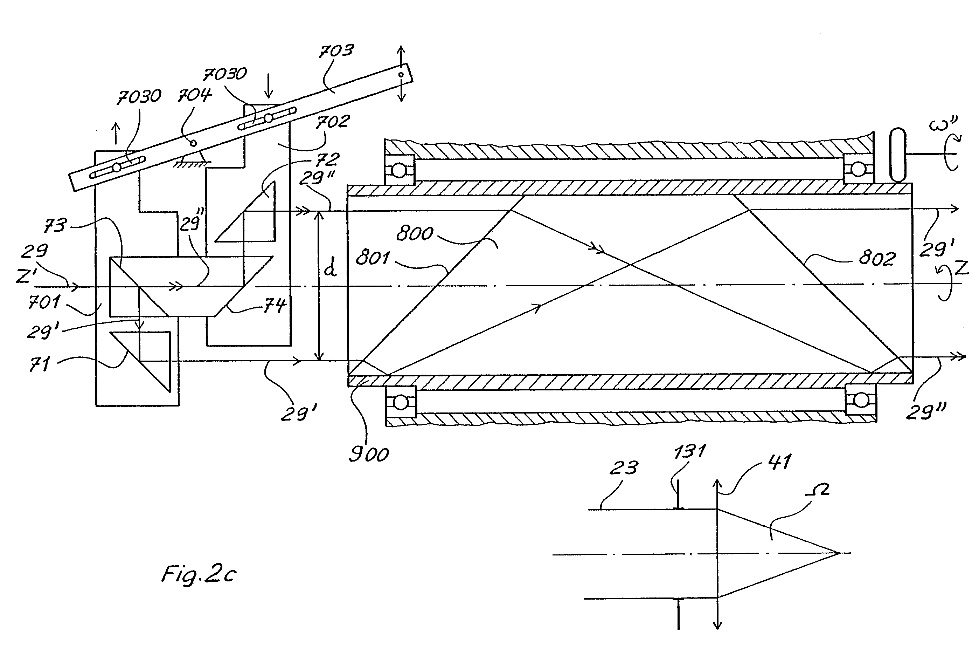

Ainsi, en tournant le tube 33, on provoque une rotation des faisceaux d'éclairage 13', 13" et de visée 29', 29" autour de l'axe optique. Il est donc possible de circonscrire et visualiser entièrement le volume de l'espace ou angle solide occupé par le faisceau laser de traitement 23 avant de tirer avec ce dernier dans l'oeil et voir s'il intercepte l'iris. En outre, comme on le verra plus en détail en liaison avec la figure 2, il est également possible d'utiliser le même dispositif pour contrôler la non partition du faisceau par le verre de contact ou tout autre obstacle, en modulant l'intensité des faisceaux 29 et 29'.

Le tube 33 offre également la possibilité de modifier avec une grande facilité la direction d'incidence sur la zone observée des faisceaux d'éclairage 13', 13" au cas où la position particulière provoquerait une réflexion directe d'une partie de là lumière dans le faisceau d'observation 1. Il est donc extrêmement facile d'éliminer cet inconvénient. Il est à remarquer que cette rotation axiale du tube 33 ne modifie en rien le réglage de visée et de mise au point et ne concerne en aucune manière la faisceau d'observation 1, qui lui, ne rencontre que des miroirs fixes.

De plus, on remarquera également que le faisceaux d'éclairage subdivisés 13' et 13" subissent chacun un nombre pair de réflexions par les miroirs placés dans le tube mobile. Ainsi, lorsque le faisceau 13 est issu d'une fente par exemple, l'orientation de la fente est conservée en son image en 12 pour toute rotation du tube 33 sur son axe.

Sur le chemin optique du faisceau d'observation 1, le rectangle 34 dénote un inverseur d'image afin de rétablir la correspondance directionnelle entre l'image et la réalité.

Le rectangle 35 dénote un filtre de protection destiné à absorber toute radiation parasite du faisceau laser de traitement qui pourrait être renvoyée directement dans le faisceau d'observation.

L'ensemble des trois lentilles convergentes ou objectifs 36, 37 et 38 forment un relais optique. L'objectif 37 situé d'une part au foyer image de 36 et d'autre part au foyer objet de 38, conjugue les objectifs 36 et 38. Ce montage connu est utilisé ici tant pour des questions de champ de bilan énergétique (conservation de lumière sur un long parcours parallèle) que pour des questions de réduction des reflets parasites en provenance du verre de contact.

Le détecteur 39 (tel un phototransistor) permet, grâce aux pertes de réflexion de la lame interférentielle 27, de mesurer et contrôler directement le caractéristiques d'émission tant temporelles qu'énergétiques de faisceau laser de traitement.

La source d'émission laser de puissance 230 comporte également un obturateur 231 à temps d'obturation variable permettant au médecin traitant de régler le temps d'obturation et le nombre d'impulsions reçues par le patient pour un type de traitement donné. L'obturateur, représenté par un disque mobile à fentes de manière schématique, peut être constitué par tout dispositif électro-optique connu de l'homme de l'art. La fréquence de répétition de ces impulsions peut également être ajustée compte tenu du traitement à effectuer par un système de commande en fréquence 232, conformément au procédé et au dispositif de commande décrit par exemple dans le brevet US 4 039 823 au nom de la demanderesse.

Selon un mode particulier de réalisation de l'invention représentée en coupe figure 2a, les faisceaux élémentaires de lumière cohérente visible 29′, 29″ sont délivrés par un système générateur d'enveloppe du faisceau laser de puissance 23. Le générateur d'enveloppe comporte un disque 50 mobile en rotation autour de l'axe Z Z′ direction de propagation du rayonnement laser visible 29. Un premier et un deuxième prisme à faces parallèles 51, 52 jouant le rôle de lames à faces parallèles sont accolés sensiblement selon un diamètre du disque et permettent la division du faisceau de rayonnement visible 29 en deux faisceaux élémentaires 29′ et 29″. Les faisceaux élémentaires 29′ et 29″ sont transmis par des trous 501, 502 du disque 50 et espacés d'une distance d sensiblement égale au diamètre du faisceau de rayonnement laser de puissance. Les prismes 51, 52 sur les figures 2a et 2b jouent le même rôle que les miroirs 17, 18, 20, 21, de la figure 1. La rotation du disque 50 entraîné à vitesse angulaire $\omega$ par friction d'un arbre moteur 500 permet le déplacement des faisceaux élémentaires 29′ et 29″ tangentiellement à l'enveloppe du faisceau laser de puissance 23. Le générateur d'enveloppe comporte en outre un modulateur 53 de l'intensité lumineuse des faisceaux élémentaires visibles 29′ et 29″. Ce modulateur est un modulateur binaire permettant la transmission et la non transmission simultanée pour les faisceaux élémentaires 29′, 29″ respectivement. Le modulateur 53 est constitué selon le mode de réalisation non limitatif de la figure 2a par un écran fixe 531 percé de trous 5310. Les trous on un diamètre sensiblement égal ou légèrement supérieur au diamètre des faisceaux élémentaires 29′, 29″ et sont disposés sur une circonférence lieu géométrique des impacts des faisceaux élémentaires au cours de la rotation du disque 50. Les trous 5310 sont espacés d'une longueur d'arc de cercle sensiblement égale au diamètre de chaque trou, deux points diamétraux du lieu geométrique des impacts comportant respectivement un trou et une absence de trou ou réciproquement.

La figure 2b représente une variante de réalisation du modulateur. Selon la figure 2b, le modulateur 53 est constitué par un disque polariseur 60 en rotation autour de l'axe Z Z′ de propagation du faisceau laser 29. Le disque polariseur 60 est par exemple entraîné par friction par l'arbre d'un moteur 600 à une vitesse angulaire $\omega'$ et transmet le faisceau visible 29 en lumière polarisée. La direction de polarisation, donnée par la flèche 60f, est variable à la vitesse $\omega'$ dans un plan perpendiculaire à l'axe ZZ′. Le modulateur comporte en outre un premier et un deuxième analyseur 510 et 520 solidaires du disque 50 et transmettant les faisceaux élémentaires 29′, 29″ respectivement en lumière polarisée suivant deux directions 510f, 520f perpendiculaires. Dans ce cas la modulation de l'intensité lumineuse des faisceaux élémentaires est effectuée à une fréquence proportionnelle à la vitesse de rotation relative $(\omega - \omega')$ du disque 50 et du polariseur 60.

Les deux types de modulateurs précités permettent la détection par l'opérateur de la coïncidence, au point de focalisation, des faisceaux élémentaires visibles 29′ et 29″ selon le fonctionnement suivant explicité en I et II figure 2b: l'intersection par un plan P 1 des faisceaux 29′ et 29″ en dehors du plan de focalisation de la lentille objectif 41, tel que représenté en I, donne naissance à deux images F′ et F″, intersection des faisceaux et du plan, clignotant à une fréquence $F_c$ liée à la vitesse de rotation $\omega$ du disque. Le déplacement relatif du plan pour l'obtention d'une intersection en P 2 au point de focalisation commun ainsi que représenté en II donne naissance à une image unique en F superposition des deux faisceaux 29′ et 29″ et exempte de clignotement. Le mode de réalisation de la figure 2b permet une optimalisation du choix des vitesses de rotation $\omega$ et $\omega'$ du disque et de l'analyseur pour un effet de détection maximal par l'opérateur. La vitesse de rotation $\omega$ peut en effet être choisie essentiellement compte tenu du temps moyen de réaction de l'opérateur pour le maintien du verre de contact sur le globe oculaire du patient, temps pendant lequel les faisceaux élémentaires 29' et 29" sont alors réputés effectuer un demi-tour du lieu géométrique de l'impact et permettent à l'opérateur de contrôler l'absence de partition du faisceau par un obstacle quelconque. La vitesse de rotation ω' peut alors être choisie par l'opérateur lui-même, en fonction de la vitesse de rotation ω précédente déjà fixée, pour un optimalisation de la sensibilité de détection.

Selon une autre variante de réalisation représentée figure 2c, la lentille de focalisation 41 du faisceau laser de puissance 23 est associée à un diaphragme ajustable 131 par exemple un diaphragme du type iris. Ce diaphragme permet de choisir l'angle solide Ω de focalisation du faisceau laser de puissance de traitement sans toutefois nécessiter de changement de focale de cette lentille suivant le diagnostic du médecin et le type d'affection à traiter. Dans ce cas, le générateur d'enveloppe est modifié de la manière ci-après selon figure 2c afin que les faisceaux élémentaires 29' et 29" demeurent représentatifs de l'enveloppe du faisceau laser de puissance quelle que soit l'ouverture du diaphragme 131. Les prismes à faces parallèles 51, 52 sont remplacés par des miroirs 71, 72 montés respectivement sur un cadre mobile 701, 702 et associés respectivement à un miroir semi-réfléchissant 73 et à un miroir 74. Le miroir semi-réfléchissant 73 et le miroir 74 sont accolés en vis-à-vis et permettent la division du faisceau laser visible 29 en deux faisceaux 29' et 29" respectivement perpendiculaires au faisceau incident 29. Le miroir 71 et le miroir 74 sont mobiles en translation suivant cette direction perpendiculaire au faisceau 29. Dans ce but les miroirs 71 et 72 sont solidaires des cadres mobiles 701, 702. La translation symétrique, perpendiculairement par rapport à la direction du faisceau laser visible 29, des cadres mobiles 701, 702 est assurée par un levier 703 à glissière 7030 mobile autour d'un point fixe 704. Les faisceaux laser visibles 29' et 29" sont renvoyés dans une direction parallèle à celle du faisceau 29 par les miroirs 71 et 72. Le déplacement du levier 703 entraîne la diminution ou l'augmentation symétrique de la distance *d* séparant le faisceaux laser visibles 29' et 29".

Le générateur d'enveloppe comporte un prisme de Dove 800 dont les faces obliques 801 et 802 jouent respectivement le rôle de face d'entrée et de sortie des rayons laser visibles 29', 29". Le prisme est monté dans un cadre mobile 900 animé par rapport à la direction de propagation du faisceau laser visible 29 d'un mouvement de rotation de vitesse angulaire ω" donnée. Une rotation du prisme d'une angle ∝ dans un premier plan perpendiculaire à la direction des faisceaux 29' et 29" entraîne une rotation de ces mêmes faisceaux en aval de la face oblique 802 de sortie des rayons d'un angle 2∝ dans un plan parallèle au premier plan. Le dispositif d'entraînement et de commande de rotation du prisme 800 et du cadre mobile 900 ne sont pas représentés pour ne pas surcharger la figure 2c. Un modulateur 53 constitué soit par un écran percé de trous comme dans le cas de la figure 6a et recevant le faisceaux 29' et 29" en aval de la face du prisme 802, ou un modulateur constitué par un polariseur 60 et des analyseurs 510, 520 peuvent sans inconvénient être utilisés avec ce type de générateur d'enveloppe.

## Revendications

1. Dispositif pour l'observation en vue du traitement de l'oeil comprenant des moyens (2 à 10 et 34 à 38) pour observer un point d'observation (12) dans l'oeil éclairé par une source lumineuse (14), des moyens (230 et 24 à 27) pour former un faisceau laser (23) de traitement par impulsions et pour le focaliser dans la zone d'observation et des moyens (17 à 22 et 28) pour former avant la mise en oeuvre du faisceau de traitement au moins deux faisceaux élémentaires (29' et 29") de lumière cohérente visible destinés à visualiser par leur intersection le point de focalisation du faisceau de traitement, caractérise en ce qu'en outre lesdits faisceaux élémentaires de lumière cohérente visible décrivent l'enveloppe du faisceau laser de traitement sur son trajet entre le dispositif et ledit point d'observation et en ce que leur intensité est modulée de manière que l'intersection du faisceau de traitement par un obstacle situé sur ledit trajet est simulée par le faisceau de lumière cohérente visible et conduise en cas d'intersection de chacun des deux faisceaux de lumière cohérente visible par ledit obstacle à un clignotement visible au niveau dudit point de focalisation.

2. Dispositif selon la revendication 1, caractérisé en ce qu'il comporte des moyens générateurs d'enveloppe (33) pour former, à partir d'une source de lumière cohérente visible (28), deux faisceaux parallèles (29', 29") dont l'écartement correspond au diamètre du faisceau de traitement, et qui tournent autour de l'axe de ce dernier à sa sortie du dispositif.

3. Dispositif selon la revendication 2, caractérisé en ce que les moyens générateurs d'enveloppe comportent un disque (50) mobile en rotation autour de l'axe du rayonnement (29) émis par la source de lumière cohérente visible, le disque comportant au moins deux trous (501, 502), et un premier et un deuxième prismes à faces parallèles (51, 52) accolés sensiblement selon un diamètre du disque mobile et permettant la division d'un faisceau de rayonnement visible en deux faisceaux élémentaires parallèles, espacés d'une distance sensiblement égale au diamètre du faisceau de rayonnement laser de traitement.

4. Dispositif selon l'une des revendication 1 à 3, caractérisé en ce qu'il comprend un modulateur de l'intensité lumineuse des faisceaux élémentaires de lumière cohérente visible constitué par un écran fixe (531) percé de trous (5310) disposés sur une circonférence lieu géométrique des impacts des faisceaux élémentaires, deux trous consécutifs étant espacés par une longueur d'arc de cercle sensiblement égale au diamètre de chaque trou, et deux points diamétraux quelconques de la circonférence comportant respectivement un trou et une absence de trou.

5. Dispositif selon l'une des revendications 2 ou 3, caractérisé en ce qu'il comprend un modulateur de l'intensité lumineuse des faisceaux élémentaires de lumière cohérente visible constitué par un disque polariseur (60) mobile en rotation autour de l'axe du rayonnement (29) émis par la source de lumière cohérente visible et placé sur la trajectoire de ce dernier, et deux analyseurs (510, 520), chacun disposés perpendiculairement à l'autre sur la trajectoire d'un des faisceaux élémentaires, les vitesses de rotation du disque polariseur autour de son axe et des faisceaux élémentaires autour de l'axe du faisceau de traitement étant différentes.

**Patentansprüche**

1. Vorrichtung zur Beobachtung des Auges im Hinblick auf seine Behandlung mit Mitteln (2 bis 10 und 34 bis 38) zum Beobachten eines durch eine Lichtquelle (14) beleuchteten Beobachtungspunktes (12) im Auge, mit Mitteln (230 und 24 bis 27) zum Bilden und Fokalisieren in die Beobachtungszone eines Laserstrahls (23) für die Behandlung durch Impulse, und mit Mitteln (17 bis 22 und 28) zum Bilden vor der Inbetriebnahme des Behandlungsstrahls mindestens zweier Elementarstrhlen (29' und 29") aus kohärentem sichtbarem Licht, bestimmt zum visuellen Kenntlichmachen des Fokalisationspunktes des Behandlungsstrahls infolge ihrer Intersektion, dadurch gekennzeichnet, dass darüber hinaus die Elementarstrahlen aus kohärentem sichtbarem Licht die Hülle des Behandlungslaserstrahls auf seiner Bahn zwischen der Vorrichtung und dem Beobachtungspunkt beschreiben, und dass ihre Intensität derart moduliert wird, dass die Intersektion des Behandlungsstrahls durch ein in dieser Bahn befindliches Hindernis durch den Strahl aus sichtbarem kohärentem Licht simuliert wird und im Falle einer Intersektion jeden der beiden sichtbaren kohärenten Lichtstrahlen durch das Hindernis zu einem sichtbaren Blinken am genannten Fokalisationspunkt führt.

2. Vorrichtung nach Anspruch 1, dadurch gekennzeichnet, dass sie Hüllengeneratormittel (33) umfasst, um zwei parallele Strahlen (29', 29") aus einer Quelle sichtbaren kohärenten Lichtes (28) zu bilden, deren Abstand dem Durchmesser des Behandlungsstrahls entspricht und die um die Achse des letzteren am Ausgang der Vorrichtung rotieren.

3. Vorrichtung nach Anspruch 2, dadurch gekennzeichnet, daß die Hüllengeneratormittel eine Scheibe (50) umfassen, die um die Achse der von der Quelle sichtbaren kohärenten Lichtes erzeugten Strahlung drehbar ist und mindestens zwei Löcher (501, 502) aufweist sowie ein erstes und ein zweites Prisma mit parallelen Seiten umfassen, die im wesentlichen längs eines Durchmessers der drehbaren Scheibe angeordnet sind und die Aufteilung eines sichtbaren Strahls in zwei parallele Elementarstrahlen ermöglichen, die voneinander einen Abstand im wesentlichen gleich dem Durchmesser des Behandlungslaserstrahles besitzen.

4. Vorrichtung nach einem der Ansprüche 1 bis 3, dadurch gekennzeichnet, daß sie einen Lichtintensitätsmodulator umfaßt für die sichtbaren kohärenten Elementarlichtstrahlen, gebildet von einem festen Schirm (531), der Lochperforationen (5310) auf einem Umfang, entsprechend dem geometrischen Ort des Auftreffens der Elementarstrahlen aufweist, wobei zwei aufeinanderfolgende Perforationen durch eine Kreisbogenlänge voneinander getrennt sind, die im wesentlichen gleich dem Durchmesser jeder Perforation ist und wobei zwei jeweils zueinander diametrale Punkte auf dem gesamten Umfang jeweils eine Perforation bzw. eine nichtperforierte Stelle aufweisen.

5. Vorrichtung nach Anspruch 2 oder 3, dadurch gekennzeichnet, daß sie einen Lichtintensitätsmodulator für die sichtbaren kohärenten Elementarlichstrahlen aufweist, gebildet von einer Polarisationsscheibe (60), die um die Achse der von der Quelle sichtbaren kohärenten Lichtes ausgehenden Strahlung drehbeweglich ist und auf der Bahn des letzteren angeordnet ist sowie zwei Analysatoren (510, 520), jeweils senkrecht zur anderen angeordnet auf der Bahn eines Elementarstrahls, wobei die Drehgeschwindigkeiten der Polarisationsscheibe um ihre Achse und der Elementarstrahlen um die Achse des Behandlungsstrahls unterschiedlich sind.

**Claims**

1. Observation apparatus for use in eye treatment comprising means (2 to 10 and 34 to 38) for observing a point of observation (12) in an eye illuminated by a light source (14), means (230 and 24 to 27) for forming a pulsed laser treatment beam (23) and for focussing said beam in the region under observation and means (17 to 22 and 28) for forming before the treatment beam is made to act at least two elementary beams (29' and 29") of visible coherent light for visualising through their intersection the focussing point of the treatment beam, characterized in that the elementary beams of visible coherent light further describe the envelope of the laser treatment beam along its path of travel between the apparatus and said point of observation and in that their intensity is modulated in such manner that any intersection of the treatment beam by an obstacle along said path of travel is simulated by the visible coherent light beam and leads in the event of intersection of each visible coherent light beam by said obstacle to visible flickering at said focussing point.

2. Apparatus as in claim 1, characterized in that it comprises envelope generating means (33) for forming, from a source (28) of visible coherent light, a pair of parallel beams (29', 29") whose spacing corresponds to the diameter of the treatment beam and which rotate about the axis of the latter as it issues from the apparatus.

3. Apparatus as in claim 2, caracterized in that the envelope generating means include a disk (50) rotatably movable about the axis of the radiation (29) emitted by the source of visible coherent light, the disk having at least two apertures (501, 502), and include first and second prisms (51, 52) having parallel surfaces, said prisms being mounted substantially along a diameter of the movable disk and enabling a beam of visible radiation to be divided into two parallel elementary beams spaced apart by a distance substantially equal to the diameter of the laser treatment beam.

4. Apparatus as in any of claims 1 to 3, characterized in that it comprises means for modulating the luminous intensity of the elementary beams of visible coherent light consisting of a stationary screen (531) formed with apertures (5310) along a loci circumference of the impacts of the elementary beams, pairs of consecutive apertures being spaced apart by a circular arc having a length substantially equal to the diameter of each aperture, and any pair of diametral points along the circumference corresponding respectively to an aperture and the absence of an aperture.

5. Apparatus as in either of claims 2 and 3, characterized in that it comprises means for modulating the luminous intensity of the elementary beams of visible coherent light consisting of a polarising disk (60) rotatably movable about the axis of the radiation (29) emitted by the source of visible coherent light and located along the path of the latter, and a pair of analysers (510, 520) each disposed at right angles to each other along the path of one of the elementary beams, the rotational speeds of the polarising disk about its axis and of the elementary beams about the axis of the treatment beam being different.

Fig. 1

Fig. 2a

Fig. 2b

2

Fig.2c

3